# EUROPEAN PATENT APPLICATION

(11) **EP 1 444 987 A2**
(43) Date of publication of application: **11.08.2004**
(21) Application number: 04010662.7
(22) Date of filing: 09.12.1999
(51) Int. Cl.: A61K 39/108, A61P 1/12

(54) **Oral vaccine against diarrhea**

(30) Priority: 18.12.1998 SE 9804415
(62) Divisional of application: 99964847.0
(71) Applicant: SBL VACCIN AB, 105 21 Stockholm (SE)
(72) Inventor: Carlin, Nils, 165 57 Hässelby (SE); Askelöf, Per, 191 41 Sollentuna (SE); Bjare, Ulf, 757 57 Uppsala (SE)
(74) Representative: Woods, Geoffrey Corlett

(57) **Abstract**

An oral vaccine composition against enterotoxigenic *E*. *coli* caused diarrhea in humans is disclosed. It comprises a defined amount of at least three different types of colonization factor antigens (CFAs), e.g. 100 to 300 µg of each type, selected from the group consisting of CFA I, CFA II (CS1, CS2 and CS3) and CFA IV (CS4, CS5 and CS6), on killed *E*. *coli* bacteria lacking the gene encoding the heat labile enterotoxin (LT⁻), together with a defined amount of the B-subunit of cholera toxin (CTB), e.g. 0.5-2.0 mg, and a vehicle, such as PBS, which vaccine composition is purified from possible heat stable enterotoxin (ST).

## Description

The present invention relates to an oral vaccine against diarrhea. More precisely, it relates to an oral vaccine composition against enterotoxigenic *E.coli* caused diarrhea in humans.

### Background

Bacteria and viruses often express on or within their membrane, proteins which in a certain environment function as a support for the organism to associate in a specific way or adhere to a surface. Such a surface may be the inner wall of the gastrointestinal tract, the oesophagus or any other biological surface or membrane in a human or animal body, where an organism may find an optimal environment for growth. Microbial membrane proteins of this kind are often named colonization factor antigens (CFA) or fimbriae. In the literature, other words such as pili, hemagglutinins, filamentous or fibrillar proteins are used for the same kind of substances. For convenience, "CFA" will be used herein to cover all these kinds of membrane proteins which also may have an antigenic character. For most organisms the production of their CFAs is controlled by various factors in the environment, which in turn may activate regulatory genes in the plasmid DNA.

A number of bacteria which are of medical interest have been shown to produce CFAs associated with their membrane. Among these, the ones which are most important for humans, are organisms which colonize the human gastrointestinal tract and the respiratory airways. Examples of organisms which colonize the gastrointestinal tract are enterotoxigenic *Escherichia coli, Vibrio cholerae, Helicobacter pylori, Campylobacter, Shigellae, Salmonellae and Yersinia.*

Of particular medical interest is the enterotoxigenic *Escherichia coli* (ETEC), since it is one of the major causes of travelers diarrhea and diarrhea among children in the developing countries and accounts for more than 1 billion diarrhea episodes and at least one million deaths per year, primarily among children.

Enterotoxigenic *E. coli* are characterized in that they produce heat labile enterotoxin (LT) and/or heat stable enterotoxin (ST).

In WO 92/14487 a method for production and use of an oral ETEC vaccine is disclosed. The ETEC bacteria with CFAs and their subcomponents (CS-antigens) are grown under specified conditions. The subsequently formalin killed bacteria may then be used as an oral vaccine against the ETEC bacteria. The CFA and CS antigens will thus function as antigens in the immunological processing that will take place in the intestine.

There were problems with the scaling up of the production of ETEC bacteria having CFAs, and a solution to the'problems were disclosed in the International Patent Application WO 95/33825.

Formulations of ETEC vaccines containing the B-subunit of cholera toxin, which is antigenically similar to the heat labile enterotoxin of ETEC, and colonization factor antigens of ETEC, were shown to stimulate relevant mucosal immune responses in Swedish volunteers.(Jertborn M., et al, Vaccine, 1997; 16:255-260).

For product safety and efficacy a vaccine composition must be non-infectious and contain defined amounts of active ingredients which are the same from batch to batch.

### Description of the invention

The present invention provides a new vaccine composition which is based on LT-negative killed enterotoxigenic *E. coli* bacteria comprising defined amounts of at least three different types of colonization factor antigens, and the B-subunit of cholera toxin (CTB) which is antigenically similar to the heat labile enterotoxin of ETEC. Further, the vaccine composition is purified from possible heat stable enterotoxin (ST).

The LT- negative ETEC bacteria are either laboratory selected from naturally genetically modified strains or produced by recombinant techniques.

Possibly present heat stable enterotoxin, which is a comparatively small protein of 19 amino acid residues, is washed away during the down stream processing of the vaccine composition.

The elimination of the possibility of the bacteria to produce heat label enterotoxin and removal of the possibly present the heat stable enterotoxin adds to product safety.

The killing of the bacteria should be performed in such a way that the colonization factor antigens are substantially retained, e.g. by mild formalin inactivation.

Thus, the present invention provides an oral vaccine composition against enterotoxigenic *E.coli* caused diarrhea in humans, which comprises a defined amount of at least three different types of colonization factor antigens (CFAs) selected from the group consisting of CFA I, CFA II (CS 1, CS2 and CS3) and CFA IV (CS4, CS5 and CS6), on killed E. coli bacteria lacking the gene encoding the heat labile (LT⁻) enterotoxin, together with a defined amount of the B-subunit of cholera toxin (CTB), and an vehicle, which vaccine composition is purified from possible heat stable enterotoxin (ST).

In an embodiment of the oral vaccine of the invention the defined amount of different types of CFAs is for each type of CFA at least 100 µg, and the defined amount of CTB is at least 0.5 mg, and the vehicle is a physiologically acceptable buffer solution.

In a preferred embodiment the defined amount of different types of CFAs is for each type of CFA 100 to 300 µg, and the defined amount of CTB is 0.5 to 2.0 mg.

In a most preferred embodiment of the invention the defined amount of different types of CFAs are 200 µg of CFA/I, 200 µg of CS1, 150 µg of CS2, 200 µg of CS4 and 150 µg of CS5, and 1.0 mg of CTB, and the physiologically acceptable buffer solution is phosphate buffered saline solution.

A dose of the vaccine composition will contain approximately 10¹¹ bacteria.

The vaccine composition is administered to person as a drink in a glas of water containing carbonate buffer, such as sodium hydrogen carbonate.

### Experiments

### Determination of the amounts of the different colonization factor antigens

*E. coli* fimbriae antigens are detected by using an inhibition ELISA substantially as described by Lopez-Vidal Y., et al in the Journal of Clinical Microbiology, vol 26, 1967-1972 (1988). Briefly, the bacterial sample to be analyzed is incubated together with a fixed amount of monoclonal antibody directed against the CFA. After incubation the mixture is transferred to a plate previously coated with CFA, where residual monoclonal antibody can bind to the CFA. Bound antibody is visualized by adding horseradish peroxidase conjugated anti-mouse immunoglobulin. As substrate, o-phenylenediaminedihydrochloride in a citrate buffer is used. The optical density is measured at 450 nm in a spectrophotometer connected to a computer equipped with a data reduction software. The optical density observed is inversely proportional to the concentration of antibody in the sample.

### Formulation of the vaccine composition

Lot of formulated oral ETEC Vaccine derived from monovalent bulks of formalin killed CFA/I SBL strain 101, CS1 SBL strain 106, CS2 SBL strain 107, CS4 SBL strain 104, CS5 SBL strain 105 respectively and monovalent bulk of purified rCTB.

| Composition | | |
|---|---|---|
| Names of Ingredients | Unit | Function |
| Active substances: | | |
| 1. Monovalent Bulk: *E.coli,* CFA/I SBL-101, Formalin Killed | 2.0 x 10² µg fimbriae | |
| | | |
| 2. Monovalent Bulk: *E.coli,* CS1 SBL-106, Formalin Killed | 2.0 x 10² µg fimbriae | To elicit immune response against ETEC bacteria |
| | | |
| 3. MonovalentBulk:*E.coli*, CS2 SBL-107, Formalin Killed | 1.5 x 10² µg fimbriae | |
| | | |
| 4. Monovalent Bulk: *E.coli,* CS4 SBL- 104, Formalin Killed | 2.0 x 10² µg fimbriae | |
| | | |
| 5. Monovalent Bulk: *E.coli,* CS5 SBL-105, Formalin Killed | 1.5 x 10² µg fimbriae | |
| | | |
| 6. Monovalent Bulk: rCTB, Purified | 1.0 mg | |
| | | |
| Vehicle: 7. PBS(WHO) Buffer, pH 7.2-7.4 | q.s. ad 6.0 ml | Buffer |

The strains SBL-104 and SBL-105 also produced CS 6, but the amount thereof was not established. The strain SBL-107 also produced CS3, but the amount thereof was not established.

### Pilot study evaluating the efficacy and reactogenicity of the oral ETEC B-subunit-killed whole cell vaccine of the invention against travelers diarrhea

In a double blind, randomized, placebo controlled pilot study three groups of Austrian travelers were investigated. In addition to the ETEC vaccine an oral B-subunit whole cell cholera vaccine was also included, since it had previously been shown in both Bangladesh ( Clemens J., et al, J Infect Dis, 1988, 158:372-377) and in Finnish travelers to Morocco (Peltola H., et al Lancet, 1991, 338:1285-89) to afford significant protection against *E.coli* LT-diarrhea.

A total of 250 volunteers, adults and children, who had signed up for a trip to tropical or subtropical destinations (44 different countries in Africa, Asia and Latin-America) with a duration of stay intended to last 7 to 23 days, were included. Twelve of these were withdrawn due to violation of protocol, so that the intent to treat population encompassed 238 persons and the per protocol population, which perfectly fulfilled all the requirements, comprised 187 travelers. Two consecutive doses of vaccine or placebo were given, at an interval of 7-21 days, not less than 7 days and not more than 30 days before departure. Post vaccination symptoms and adverse events were reported after both doses. All volunteers enrolled in the study were equipped with a daily record diary to monitor episodes of travelers' diarrhea during their stay abroad and with transport media (Portagerm and Cary Blair tubes) for collection of stool samples in case of diarrhea. Travelers were instructed to hand over their travel diary, and transport media tubes immediately after return. Blood samples for testing immunogenicity were taken from a subset of 73 volunteers before and after the first and second dose of the vaccines or placebo. The post first dose. sample was collected immediately before intake of the second dose, and the post second dose blood sample was drawn after return.

The formulations given to the three randomized groups of travelers were: ETEC vaccine., containing 1 mg of recombinant B-subunit of cholera toxin plus 10¹¹ formalin killed ETEC bacteria of five ETEC strains expressing the most common colonization factor antigens (CFA's) such as CFA I, CFA II (CS 1, CS2 and CS3) and CFA IV (CS4, CS5 and CS6); a B-subunit cholera whole cell vaccine (registered in Sweden since 1992), containing 1 mg recombinant subunit B cholera toxin and 10¹¹ killed whole cells (Inaba, Ogawa, classical and El Tor); placebo containing 10¹¹ killed *E.coli* K12. The formulations were suspended in 4 ml buffer and each dose of vaccine or placebo was given as a drink in 150 cc of a sodium hydrogen carbonate solution. Isolation and characterization of ETEC bacteria and the anti-CTB IgA ELISA were performed as described (Jertborn M., et al ibid).

250 volunteers received one dose of vaccine or placebo, of whom 246 also received the second dose. The study protocol was approved by the local. Ethics Committee before start of the study. Written informed consents of the volunteers were obtained before randomization.

Reactogenicity results are as follows: In total 43 volunteers (17%) had mild to moderate gastrointestinal or general symptoms after the first. dose; 13 (16%) in the placebo, 13 (16%) in the cholera vaccine group and 17 (20%) in the ETEC vaccine group. After the second dose 20 (8%) had symptoms; 6 (7%) in the placebo, 7 (9%) in the cholera vaccine group and 7 (8%) in the ETEC vaccine group. Statistically, there were no differences in the proportion of volunteers reporting symptoms between the study groups.

Evaluating the efficacy of the vaccines, a case of ETEC diarrhea was defined as having three or more liquid stools, and ETEC, but no other pathogens, detected in pretreatment stools. In the Per Protocol population the incidence of ETEC was 8%(5 cases) in the placebo group, 10%(6 cases) in the cholera vaccine group and 2% (1 case) in the ETEC vaccine group. The vaccine efficacy as regards ETEC vaccine versus placebo was 79% (p=0.119) and versus cholera vaccine 84% (p=0.0496). Comparison of ETEC diarrhea rates was done by one-sided Fisher's exact test.

All ETEC isolates in the Per Protocol population expressed colonization factors included in the vaccine.

The cholera vaccine gave no detectable protection against ETEC as compared with the placebo group, but it is noteworthy that only two of the ETEC cases in the cholera vaccine group were caused by bacteria producing solely the heat-labile enterotoxin (LT) against which the cholera vaccine (through its B-subunit) can theoretically work.

Thus, due to the fact that there were almost exclusively LT-negative strains present in this group, the cholera vaccine is not supposed to have any effect.

Despite these results it must be kept in mind that the stool sample handling as described might be a source of error. When using transport media, there is a risk of losing sensitive pathogens, whereas less sensitive ones such as *E. coli* bacteria survive, resulting in false positive cases of ETEC diarrhea (see definition of ETEC diarrhea). Hence, the proportion of ETEC diarrhea might have been overestimated. However, it is unlikely that possible false positive cases are unevenly distributed over the study groups.

Concerning immunogenicity, a significant increase in antibodies against the B-subunit of choleratoxin (CTB) could be shown by means of anti-CTB IgA ELISA in the group receiving the ETEC vaccine (geometric mean titres pre- vs. post-vaccination: 7.8 vs. 58.7 after the first and 62.6 after the second dose; seroconversion after 2nd dose: 91%) and in the group receiving, the cholera vaccine (7.6 vs. 54.6 after the first and 60.1 after the second dose; seroconversion 100%). The placebo group did not show any significant increase in the anti-CTB response (7.9 vs. 8.3 after the first and 11.8 after the second dose; seroconversion 29%).

Thus - although the CTB-cholera WC vaccine induced a very good response against the B-subunit - a significant influence of this vaccine on ETEC diarrhea was not observed. This could predominantly be ascribed to the fact that a majority of the ETEC strains isolated from these voluateers were only producing ST (9 from 12) and hence could not be prevented by the cholera vaccine. Apparently, anti-CFA antibodies are highly important for protection against in particular diarrhea caused by ST-only ETEC.

The present results are the first to lend support in humans for the working hypothesis that oral ETEC vaccine induced anti-CFA/CS immunity can protect against diarrhea caused by ST-only producing ETEC.

## Claims

1. Oral vaccine composition against enterotoxigenic *E.coli* caused diarrhea in humans, which comprises a defined amount of at least three different types of colonization factor antigens (CFAs) selected from the group consisting of CFA I, CFA II (CS 1, CS2 and CS3) and CFA IV (CS4, CS5 and CS6), on killed *E. coli* bacteria lacking the gene encoding the heat labile (LT') enterotoxin, together with a defined amount of the B-subunit of cholera toxin (CTB), and an vehicle, which vaccine composition is purified from possible heat stable enterotoxin (ST).

2. Oral vaccine according to claim 1, wherein the defined amount of different types of CFAs is for each type of CFA at least 100 µg, and the defined amount of CTB is at least 0.5 mg, and the vehicle is a physiologically acceptable buffer solution.

3. Oral vaccine according to claim 2, wherein the defined amount of different types of CFAs is for each type of CFA 100 to 300 µg, and the defined amount of CTB is 0.5 to 2.0 mg.

4. Oral vaccine according to claim 3, wherein the defined amount of different types of CFAs are 200 µg of CFA/I, 200 µg of CS1, 150 µg of CS2, 200 µg of CS4 and 150 µg of CS5, and 1.0 mg of CTB, and the physiologically acceptable buffer solution is phosphate buffered saline solution.
